# EUROPÄISCHE PATENTANMELDUNG

(11) **EP 4 596 012 A1**
(43) Veröffentlichungstag der Anmeldung: **06.08.2025**
(21) Anmeldenummer: 24154733.0
(22) Anmeldetag: 30.01.2024
(51) Int. Cl.: A61M 16/04, A61B 5/00

(54) **BEATMUNGSTUBUS MIT AKUSTISCHEM SENSOR**

(71) Anmelder: Georg-August-Universität Göttingen Stiftung Öffentlichen Rechts, Universitätsmedizin, 37075 Göttingen (DE)
(72) Erfinder: FRIEDRICH, Martin, 37120 Bovenden (DE)
(74) Vertreter: REHBERG HÜPPE + PARTNER

(57) **Zusammenfassung**

Die Erfindung einen Beatmungstubus (1) zum Beatmen eines Patienten, aufweisend einen, einen Luftkanal (25) umgebenden Schlauch (2) mit einer Schlauchwandung (3) zum Einführen bis in einen Rachen oder bis in eine Luftröhre des Patienten, eine Manschette (4, 15) mit einem in Umfangsrichtung der Schlauchwandung (3) umlaufenden Luftvolumen (6), eine in Axialrichtung des Schlauches (2) verlaufende Pumpleitung (5) zum Herstellen einer Strömungsverbindung zwischen dem Luftvolumen (6) der Manschette (4, 15) und einer Pumpeinrichtung (7). Um insbesondere die Atmung beziehungsweise Beatmung des Patienten betreffende Zustände in hoher Qualität detektieren zu können, wird vorgeschlagen, dass der Beatmungstubus (1) einen in dem Luftvolumen (6) auf der Schlauchwandung (3) oder einem an den Luftkanal (25) angrenzenden Teilbereich der Manschette (4, 15) angeordneten akustischen Sensor (8, 16) aufweist.

## Beschreibung

### TECHNISCHES GEBIET DER ERFINDUNG

Die Erfindung betrifft einen Beatmungstubus zum Beatmen eines Patienten, wobei der Beatmungstubus einen, einen Luftkanal umgebenden Schlauch mit einer Schlauchwandung zum Einführen bis in einen Rachen oder bis in eine Luftröhre des Patienten, eine Manschette mit einem in Umfangsrichtung der Schlauchwandung umlaufenden Luftvolumen und eine in Axialrichtung des Schlauches verlaufende Pumpleitung zum Herstellen einer Strömungsverbindung zwischen dem Luftvolumen der Manschette und einer Pumpeinrichtung aufweist.

Daneben betrifft die Erfindung ein System aus einem solchen Beatmungstubus und einer Recheneinrichtung.

Unter Beatmungstubus wird hier insbesondere ein flexibler Schlauch verstanden, welcher zur sogenannten künstlichen Beatmung eines Patienten verwendet wird, wobei der Patient ein Mensch oder ein Tier sein kann. Der Beatmungstubus kann als Kehlkopfmaske (Larynxmaske) oder als Endotrachealtubus ausgebildet sein. Bei der Anwendung wird der Beatmungstubus - je nach Ausführung - über Mund oder Nase bis in den Rachen oder bis in die Luftröhre des Patienten eingeführt.

Eine Kehlkopfmaske wird durch den Mund des Patienten in dessen Rachen eingeführt und dort vor den Kehlkopf gesetzt. Eine endseitige Manschette, welche das freie Ende des Schlauches ohrmuschelförmig umgibt, wird anschließend aufgeblasen, um den Eingang des Kehlkopfes optimal zu umschließen.

Ein Endotrachealtubus wird hingegen bis in die Luftröhre des Patienten eingeführt. Dieser Vorgang ist üblicherweise unter dem Begriff Intubation bekannt. Der Endotrachealtubus weist eine, einen axialen Abschnitt der Schlauchwandung umgebende Manschette auf, manchmal auch zwei Manschetten. Gegenüber einer Kehlkopfmaske hat der Endotrachealtubus den Vorteil, dass die Atemwege besser vor einer Aspiration, d. h. vor einem Zurückfließen von Mageninhalt über die Speiseröhre in den Mund-Rachenraum und die Luftröhre bis in das Lungengewebe, geschützt werden.

Die künstliche Beatmung des Patienten wird beispielsweise vor einer Operation, bei einer intensivmedizinischen Behandlung in einem Krankenhaus oder in einem Rettungswagen durchgeführt. Sobald der Beatmungstubus weit genug in den Körper des Patienten vorgeschoben ist, wird die Manschette mit Luft gefüllt, meist nur wenigen Millilitern Luft, die über eine handbetätigte Spritze eingeleitet werden, um den Tubus in der Luftröhre zu fixieren bzw. abzudichten. Ein zu niedriger Druck dichtet nicht zureichend ab, ein zu hoher hingegen gefährdet die Durchblutung der Schleimhaut. Es können an dem Beatmungstubus auch zwei in Längsrichtung des Schlauches hintereinander liegende Manschette vorgesehen sein, was bei zeitlich wechselnder Blockung zusätzlich die Schleimhaut entlastet. Sodann kann die Beatmung des Patienten eingeleitet werden, welche - je nach Situation - entweder über eine automatische Beatmungsmaschine, einen sogenannten Respirator, oder über einen handbetätigten Beatmungsbeutel erfolgt. Bevor der Beatmungstubus später wieder aus dem Körper des Patienten entfernt wird, muss die Luft aus der Manschette abgelassen werden.

### STAND DER TECHNIK

Ein Beatmungstubus mit den Merkmalen des Oberbegriffs des unabhängigen Patentanspruchs 1 ist aus der WO 2021/226105 A1 bekannt. Der darin beschriebene Endotrachealtubus weist eine Manschette (Cuff) mit einem oder mehreren Sensoren zur Messung eines Druckes zwischen dem Endotrachealtubus und einer Trachealwand des Patienten auf. Damit soll vermieden werden, dass die Manschette den trachealen Blutfluss beeinflusst und Komplikationen während der Intubation hervorruft. Die Manschette kann ein oder mehrere Materiallagen aufweisen, wobei der Sensor in eine Materiallage oder zwischen mehreren Materiallagen eingebettet sein kann. Als zusätzliche Sensoren sind des Weiteren Flusssensoren, CO₂-Sensoren, Ultraschallsensoren und andere Sensoren zur Messung physiologischer Parameter wie beispielsweise Blutfluss, Herzrate, Blutdruck, Herzzeitvolumen beschrieben.

Daneben ist aus der US 2016/0183819 A1 ein Katheter mit einer Einrichtung zur Detektion eines Druckes innerhalb einer Flüssigkeitskammer des Katheters bekannt, um ein Eintreten von Flüssigkeit in den Katheter zu erkennen. Es ist beschrieben, zu diesem Zweck einen mechanischen oder faseroptischen Drucksensor zu verwenden. Des Weiteren werden Körpergeräusche eines Patienten durch ein in dem Katheter (oder in einem Implantat) befindliches Mikrofon detektiert. Die Körpergeräusche können beispielsweise Sprache, Schlafapnoe, Asthma, normale oder abnormale Atemgeräusche, Darmgeräusche, Herzgeräusche und andere sein.

Des Weiteren sind aus der WO 2020/023562 A1 oder der US 2009/0025459 A1 medizinische Implantate bekannt, welche akustische Sensoren, Temperatursensoren oder Viskositätssensoren aufweisen können. Des Weiteren ist beschrieben, in Abhängigkeit von den detektierten Signalen eine automatische Therapieempfehlung zu generieren oder ein über das Implantat in den Körper eingebrachtes Medikament freizusetzten.

Auch sind nicht invasive Messmethoden beziehungsweise Sensoren im Stand der Technik beschrieben, mit welchen Parameter einer untersuchten Person detektiert werden können, zum Beispiel eine Herzfrequenz. Anhand dieser wird dann eine Diagnose einer Erkrankung getroffen. Diesbezüglich wird auf die Patentschrift EP 2 004 037 B1 hingewiesen.

Bei der erstgenannten Druckschrift, WO 2021/226105 A1, welche als einzige einen Beatmungstubus gemäß dem Oberbegriff des Anspruchs 1 betrifft, ist der Sensor in der von dem Schlauch wegweisenden äußeren Umfangsfläche der Manschette angeordnet, sodass der Sensor relativ weit von dem, von dem Schlauch umgebenen Luftkanal des Beatmungstubus entfernt ist. Der Sensor ist damit durch das Luftvolumen der Manschette von dem Ort der Luftströmung und den dort auftretenden Atem- bzw. Beatmungsgeräuschen getrennt. Insbesondere hochfrequente Geräusche werden dadurch stark gedämpft. Mit dem beschriebenen Sensor werden daher physiologische Parameter des Patienten im Wesentlichen nur dort detektiert, wo vorrangig Blutfluss, Blutdruck und Herzzeitvolumen wahrnehmbar sind. Die von dem Sensor detektierbare Amplitude der akustischen Signale, welche durch den Atemvorgang bzw. Beatmungsvorgang hervorgerufen sind, wird hingegen durch die Platzierung des Sensors stark herabgesetzt.

### AUFGABE DER ERFINDUNG

Der Erfindung liegt daher die Aufgabe zugrunde, einen Beatmungstubus sowie ein System aus einem Beatmungstubus und einer Recheneinrichtung aufzuzeigen, bei welchen Parameter der Atmung bzw. Beatmung eines Patienten mit hoher Qualität aufgenommen und analysiert werden können, insbesondere um anhand der detektierten Daten auf abnormale Vorgänge schließen zu können.

### LÖSUNG

Die Aufgabe der Erfindung wird durch einen Beatmungstubus mit den Merkmalen des unabhängigen Patentanspruchs 1 gelöst. Die abhängigen Patentansprüche 2 bis 11 beschreiben bevorzugte Ausführungsformen des erfindungsgemäßen Beatmungstubus. Der Patentanspruch 12 ist auf ein System aus einem erfindungsgemäßen Beatmungstubus und einer Recheneinrichtung gerichtet. Die abhängigen Patentansprüche 13 und 14 beschreiben bevorzugte Ausführungsformen des erfindungsgemäßen Systems.

### BESCHREIBUNG DER ERFINDUNG

Die Erfindung schlägt vor, dass ein Beatmungstubus, der einen, einen Luftkanal umgebenden Schlauch mit einer Schlauchwandung zum Einführen bis in einen Rachen oder eine Luftröhre des Patienten, eine Manschette mit einem in Umfangsrichtung um die Schlauchwandung umlaufenden Luftvolumen und eine in Axialrichtung des Schlauches verlaufende Pumpleitung zum Herstellen einer Strömungsverbindung zwischen dem Luftvolumen der Manschette und einer Pumpeinrichtung aufweist, weiterhin einen in dem Luftvolumen auf der Schlauchwandung oder einem an den Luftkanal angrenzenden Teilbereich der Manschette angeordneten akustischen Sensor aufweist.

Erfindungsgemäß befindet sich der akustische Sensor somit innerhalb des Luftvolumens, dessen Bezeichnung nicht ausschließt, dass es über die Pumpeinrichtung auch mit einem anderen Gas als Luft befüllt wird. Im Falle eines Endotrachealtubus ist das Luftvolumen zwischen der Schlauchwandung und der Manschette ausgebildet, so dass der akustische Sensor die Schlauchwandung berührt. Damit liegt der akustische Sensor an dem Schlauch an, welcher Luft von einer Beatmungseinrichtung, nämlich einer Beatmungsmaschine oder einem Beatmungsbeutel, durch den Luftkanal in den Körper des Patienten führt. Im Falle einer Kehlkopfmaske ist das Luftvolumen vollständig von dem Material der Manschette umgeben, wobei in einem Querschnitt bezogen auf eine gedachte Verlängerung des Luftkanals des Schlauches eine radial innere Innenwandung und eine radial äußere Innenwandung der Manschette definiert werden können. Der akustische Sensor liegt dabei an einem an den Luftkanal angrenzenden Teilbereich der Manschette an, nämlich an der so definierten radial inneren Innenwandung.

Durch die räumliche Nähe von Sensor und freiem Querschnitt der Luftröhre können die Geräusche der Atemwege mit hoher akustischer Qualität aufgenommen werden. Dadurch ist es möglich, viele verschiedene Atemwegszustände zuverlässig zu unterscheiden und als verlässliche Indikation für eine Behandlung des Patienten heranzuziehen. Krankheiten des Patienten beziehungsweise der Bedarf einer Behandlung können schnell, bevorzugt noch während der Beatmung, erkannt werden, ohne dass es einer weiteren, separaten Diagnostik bedarf. Anhand der direkt an der Schlauchwandung des Beatmungstubus aufgenommenen akustischen Signale können digitale Audio-Profile verschiedener Atmungszustände und/oder Beatmungszustände erstellt und unterschieden werden. So können anhand eines aufgenommenen Datensatzes, welcher beispielsweise zumindest zwei Atemzyklen umfasst, verschiedene Zustände festgestellt werden, beispielsweise ob der Beatmungstubus ordnungsgemäß innerhalb des Patienten verlegt ist, ob ein dünn- oder dickflüssiger Schleim in den Atemwegen vorhanden ist, ob der Patient hustet oder ob eine maschinelle oder spontane Atmung des Patienten vorliegt. Des Weiteren kann ein Pendelvolumen bestimmt werden, welches eine von dem Patienten ausgeatmete Luftmenge beschreibt, die in dem Beatmungstubus verbleibt und nicht durch genügend Frischluft ersetzt wurde. Somit kann anschließend verhindert werden, dass das Pendelvolumen eine kritische Schwelle übersteigt, wodurch der Patient beim nächsten Atemzyklus die bereits verbrauchte Luft wieder einatmen würde. Ebenso können die am Respirator voreingestellten Volumina und zeitlichen Abläufe von Atemzug zu Atemzug mit dem Istzustand im Beatmungstubus abgeglichen werden. Die hohe Präzision der akustischen Aufnahme erlaubt das gezielte Eingreifen in den Atem- beziehungsweise Beatmungsvorgang des Patienten durch insbesondere sofortige maschinelle oder manuelle Maßnahmen eines Behandlers.

Es wird des Weiteren vorgeschlagen, dass der akustische Sensor zumindest in expandiertem Zustand der Manschette durch das Luftvolumen von einer gegenüberliegenden radial äußeren Innenwandung der Manschette beabstandet ist. Dadurch ist bei einem expandierten Zustand der Manschette sichergestellt, dass der akustische Sensor die radial äußere Innenwandung der Manschette nicht berührt. Damit wird gezielt beeinflusst, dass der akustische Sensor vorrangig die Geräusche der Atemwege beziehungsweise der künstlichen Beatmung im Bereich des Luftkanals des Beatmungstubus wahrnimmt und nicht etwa die Geräusche, die an der Außenwandung der Manschette präsent sind und andere Körperfunktionen des Patienten betreffen, beispielsweise dessen Herzschlag oder Verdauung. Durch die Platzierung des erfindungsgemäßen akustischen Sensors ohne Kontakt zu der radial äußeren Innenwandung der Manschette können die akustischen Signale in einem luftgefüllten Raum, nämlich in dem Luftvolumen der Manschette, aufgenommen werden. Durch die Anordnung des Sensors auf der Schlauchwandung (bei einem Endotrachealtubus) bzw. auf einem an den Luftkanal angrenzenden Teilbereich der Manschette (bei einer Kehlkopfmaske) und die damit geschaffene räumliche Nähe zu dem Luftkanal des Schlauches werden somit vorrangig die dort auftretenden Atmungsbeziehungsweise Beatmungsgeräusche erfasst.

Es wird vorgeschlagen, dass der akustische Sensor ein Mikrofon ist. Insbesondere kann der akustische Sensor ein als Komponente eines Mikrosystems ausgebildetes Mikrofon, das heißt ein sogenanntes MEMS-Mikrofon, sein, wobei die Abkürzung MEMS für ein mikro-elektromechanisches System steht. Die Signale des Mikrofons können zunächst durch einen Mikrofonverstärker verstärkt werden und anschließend mittels eines Analog-Digital-Wandlers in ein digitales Audioformat überführt werden. Zusätzlich kann auch ein Piezo-Sensor oder Gyrosensor wertvolle Zusatzinformationen liefern, die beispielsweise durch eine Lageveränderung des Patienten (zum Beispiel Sitzen oder Liegen) verursacht werden. Die dadurch entstehenden Signale bzw. Signaländerungen können ebenso verwertet werden. Die Abspeicherung der digitalen Signale kann in Form unterschiedlicher Dateiformate erfolgen, beispielsweise in Form sogenannter Wave-Formate. Vorzugsweise wird ein apparativ bedingtes Grundrauschen aus den aufgenommenen akustischen Daten herausgefiltert. Dabei kann gezielt ein bestimmtes Signal-Rauschverhältnis, sogenanntes signal noise ratio (SN-Verhältnis), angestrebt werden. Die gespeicherten Daten können einem Behandler, insbesondere einem Arzt, zur Kenntnis gebracht werden, beispielsweise als akustisches Signal, z. B. auf einem Stethoskop, oder als optisches Signal in Form von Text und/oder Bildzeichen auf einem Bildschirm oder mittels einer farblich kodierten Leuchtanzeige. Daraufhin kann der Behandler Maßnahmen für die weitere Behandlung ergreifen. Darüber hinaus können die Audiodaten einer maschinellen Analyse unterzogen werden, um Zustände der Atmung oder Beatmung beziehungsweise des Patienten zu erkennen. Die Analyse kann besondere mathematische Methoden, beispielsweise eine FFT-Analyse, umfassen. Des Weiteren können v.a. bei bewusstlosen beatmeten Menschen und Tieren die regelmäßigen, durch den Respirator erzeugten Strömungsmuster digital aufsummiert werden, so dass sich unregelmäßige Artefakte gegenseitig rechnerisch aufheben und damit das SN-Verhältnis weiter verbessern. Mittels künstlicher Intelligenz kann beispielsweise eine Undichtigkeit des Beatmungstubus oder eine besondere Atemsituation des Patienten, beispielsweise Husten, Spastik, Schleimbildung innerhalb der Atemwege und anderes, erkannt werden.

Es können auch mehrere Mikrofone eingesetzt werden, beispielsweise um innerhalb desselben Körperbereiches des Patienten ein Stereosignal oder 3D-Audio bzw. mehrdimensionales Audiomuster aufzunehmen. Damit lässt sich durch Detektion und Berechnen von Laufzeitunterschieden eine festgelegte Hörrichtung bevorzugen. Es können auch entferntere Signale wie Darmgeräusche oder Herzgeräusche gezielt unterdrückt werden, wobei diese aber dennoch erkannt werden können, um sie medizinisch verwerten zu können. Mehrere Mikrofone können dabei über eine gemeinsame Schnittstelle gekoppelt sein, um detektierte Signale gemeinsam an eine Recheneinrichtung weiterzuleiten. Dies reduziert den Aufwand für elektrische Leitungen bzw. Signalleitungen an dem Beatmungstubus.

Sofern das Mikrofon als MEMS-Mikrofon ausgebildet ist, ist eine Platzierung des Mikrofones oder mehrerer Mikrofone auf einer Platine möglich, auch um ein 3D- oder mehrdimensionales Wahrnehmen zu ermöglichen. MEMS-Mikrofone weisen üblicherweise zwei Luftkammern auf, die durch eine flexible Membran getrennt sind. Die flexible Membran kann durch Schalldruck verlagert werden, wodurch sich eine Kapazität des Bauteils ändert, die als elektrische Spannungsänderung detektiert werden kann. Die Umwandlung des Schallsignals in ein Spannungssignal erfolgt aufgrund einer Änderung einer elektrischen Kapazität zwischen einer feststehenden Grundplatte einer der Luftkammern des Mikrofons und der flexiblen Membran. Die Kapazitätsänderung wird durch die angreifenden Schallwellen ausgelöst, die durch eine Schalleintrittsöffnung der entsprechenden Luftkammer auf die Membran gelangen und diese dann verformen, wodurch sich das freie Volumen zwischen der Grundplatte der Luftkammer und der Membran ändert.

Bevorzugt weist sowohl die erste Luftkammer, als auch die zweite Luftkammer eine Strömungsverbindung zu dem Luftvolumen auf. Gemäß dieser Ausgestaltung verfügt das Mikrofon über zwei Luftkammern, welche relativ zu dem Luftvolumen offen ausgebildet sind. Dies sorgt für die Funktionsfähigkeit des Mikrofons innerhalb des Überdrucks der aufgeblasenen Manschette und sichert den maximal weiten Frequenzgang des Mikrofones. Anderenfalls wäre das Mikrofon bei geschlossener zweiter Luftkammer nicht funktionsfähig, da der Überdruck des Luftvolumens der Manschette zu einem statischen Differenzdruck zwischen der offenen und der geschlossenen Luftkammer des Mikrofons führen würde, der viel größer als die zu erfassenden Schalldrücke ist und der die Membran stark statisch auslenken oder sogar zerstören würde. Wie vorgeschlagen steht somit sowohl an der ersten Luftkammer, als auch an der zweiten Luftkammer die Überdruckatmosphäre der Manschette an und sorgt dafür, dass sich die Membran in Abhängigkeit des in die erste Luftkammer eintretenden Schalls verformen kann.

Gemäß einer möglichen Ausführungsform ist vorgesehen, dass die Pumpleitung in die Schlauchwandung integriert ist. Die zum Aufpumpen des Luftvolumens der Manschette dienende Pumpleitung ist somit ein integraler Bestandteil des Schlauches selbst und muss nicht separat neben dem Schlauch bereitgestellt werden. Dies schützt die Pumpleitung vor einer Beschädigung und sorgt für ein zuverlässiges Expandieren der Manschette. Damit behält der Schlauch eine möglichst zylindrische äußere Form, sodass der Beatmungstubus möglichst optimal innerhalb der Luftröhre des Patienten liegt.

Des Weiteren kann vorgesehen sein, dass eine elektrische Leitung für den akustischen Sensor in die Schlauchwandung integriert ist. Gleiches wie zuvor für die Pumpleitung der Manschette ausgeführt, kann somit auch für die elektrische Leitung des akustischen Sensors gelten. Die in das Material der Schlauchwandung integrierte elektrische Leitung verläuft somit geschützt in bevorzugt axialer Richtung innerhalb der Schlauchwandung. Die elektrische Leitung ist damit nicht zuletzt vor einer mechanischen Beanspruchung geschützt, was wiederum die Funktionsfähigkeit des akustischen Sensors sicherstellt und ebenso auch mechanische Alterationen der Schleimhaut verhindert.

Der Sensor oder Sensoren können auch so ausgebildet sein, dass diese ohne Kabelanbindung, beispielsweise über Bluetooth oder andere digitale bzw. analoge Funksignale, Informationen an einen Empfänger weitergeben.

Neben dem akustischen Sensor kann der Beatmungstubus einen oder mehrere weitere Sensoren zur Detektion weiterer physiologischer Parameter aufweisen. Der eine weitere Sensor oder die mehreren weiteren Sensoren können einerseits ebenfalls akustische Sensoren zur Detektion von Atemgeräuschen oder Beatmungsgeräuschen sein, andererseits aber auch ausgebildet sein zur Detektion von Parametern wie Blutfluss, Herzfrequenz, Atemfrequenz, Blutdruck, Temperatur, Druck. Zur Detektion einiger der vorgenannten Parameter, insbesondere des Blutflusses, der Herzfrequenz, des Blutdruckes, bietet es sich an, dass der weitere Sensor innerhalb des Luftvolumens der Manschette an einer radial äußeren Innenwandung der Manschette oder ganz außerhalb des Luftvolumens der Manschette positioniert ist. Bei einer solchen Positionierung liegt der weitere Sensor bevorzugt dort, wo die vorgenannten Parameter besonders gut gemessen werden können. Insofern können mittels des Beatmungstubus eine Vielzahl von Körpergeräuschen detektiert werden, ohne dass zusätzliche separate Untersuchungen vorgenommen werden müssen. Der Beatmungstubus, welcher ohnehin zur Beatmung des Patienten verwendet wird, beispielsweise während einer Operation oder eines Notfalls, dient somit gleichzeitig zum Einführen einer Vielzahl unterschiedlicher Sensoren, welche Aufschluss nicht nur über Atemgeräusche oder Beatmungsgeräusche des Patienten geben können, sondern vielmehr auch über Parameter des Herzens, des Magens, des Darms oder andere. Die weitere Belastung des Patienten durch zusätzliche separate Untersuchungsmaßnahmen wird somit erheblich reduziert. Es ist nicht erforderlich, in weiteren Arbeitsschritten andere Sensoren in den Körper einzuführen. Anhand des akustischen Profils des Patienten sowie des aus weiteren physiologischen Parametern bestehenden Profils, beispielsweise betreffend Herz, Darm, Magen, kann die körperliche Gesamtkonstitution des Patienten festgestellt werden. Die Auswertung der Daten kann entweder maschinell anhand von Vergleichsdaten vorgenommen werden oder manuell durch einen Arzt oder medizinisches Fachpersonal erfolgen. Außerdem können selbstlernende Algorithmen ein digitales Sensordaten-Abbild generieren, welches in geschlossenen Regelsystemen sich selbst verbessernd eine Rolle spielt.

Des Weiteren können optische Sensoren zum Einsatz kommen. Die Wand des Tubus kann dafür transparent gestaltet sein, so dass berührungsfrei nach innen (optische Beschaffenheit des Sekretes: Gelb = bakterieller Infekt, hell = viraler Infekt, rötlich = Lungenödem, Blutung, Sauerstoffgehalt, usw.) und/oder nach außen (Schleimhautdurchblutung, Sauerstoffsättigung, Elektrolyte oder andere Substanzen) detektiert werden kann.

Der Sensor kann so ausgebildet sein, dass bestimmte Frequenzen aus einem Spektrum abgegeben werden, um deren Extinktion oder Verschiebung an einem gegenüberliegenden optischen Sensor abzubilden. Diese optischen Signale können über Lichtleiterkabel oder Totalreflexionen im Beatmungstubus weitergeleitet werden oder ausgeleitet werden. Somit lassen sich optisch Substanzen, Fluidzusammensetzungen oder Bewegungen detektieren. Eine mögliche Anwendung wären Temperaturmessungen mittels Infrarotspektren mehrmals pro Atemzyklus. Ebenso ließe sich so der Kapillarpuls als Parameter der Schleimhautdurchblutung ableiten.

Des Weiteren könnte ein Sensor einen Säuregrad messen (pH-Wert). Damit ließe sich ein für das Überleben kritischer Aspirationszustand ad hoc erkennen und behandeln, indem die Manschette umgehend mit höherem Druck beaufschlagt wird und zusätzlich möglicherweise ein Spül-Saugvorgang eingeleitet würde, der einen Schaden in den tiefer liegenden Atemwegen oder der Lunge verhindert bzw. abmildert.

Die Daten können insbesondere in ein Dokumentationssystem einfließen und einer komplexen Analyse durch künstliche Intelligenz unterzogen werden. Auf der Basis einer umfangreichen Datenmenge vieler verschiedener Patienten können Therapievorschläge abgeleitet und zur besseren Diagnostik herangezogen werden. Langfristig können mittels mathematischer Methoden zuverlässig Krankheiten bzw. der Bedarf einer Behandlung aus den Metadaten vieler Patienten herausgefiltert werden, wobei Missstände unter Umständen bereits während der Datenaufnahme behoben werden können, bevor der Patient überhaupt etwas von der Erkrankung beziehungsweise Beeinträchtigung bemerkt.

Des Weiteren können nach erfolgter Diagnose medizinische Geräte angesteuert werden, um die Situation des Patienten zum Positiven zu verändern. Beispielweise kann ein Sauger angeschaltet werden, um Schleim aus den Atemwegen des Patienten abzusaugen oder ähnliches.

Es kann des Weiteren vorgesehen sein, dass der Schlauch des Beatmungstubus eine in Axialrichtung verlaufende Strömungsleitung zum Absaugen von Körperflüssigkeiten oder zum Spülen von Körperpartien aufweist. Die Strömungsleitung kann bevorzugt in die Schlauchwandung integriert sein. Gemäß der bevorzugten Ausführungsform steht die Strömungsleitung somit nicht über die äußere Kontur der Schlauchwandung hervor. Damit ist einerseits eine besonders symmetrische Form des Beatmungstubus sichergestellt und andererseits die mechanische Stabilität der Strömungsleitung innerhalb des Beatmungstubus.

Gemäß einer weiteren Ausführungsform wird vorgeschlagen, dass der Beatmungstubus zwei, die Schlauchwandung umgebende und voneinander beabstandete Manschetten aufweist, wobei ein erster akustischer Sensor einer ersten Manschette zugeordnet ist und wobei ein zweiter akustischer Sensor einer zweiten Manschette zugeordnet ist. Bei einer solchen Ausgestaltung kann eine Mehrkanalauflösung der Messdaten erreicht werden. Beispielsweise können mehrere akustische Sensoren, insbesondere Mikrofone, gleichzeitig im Körper eingesetzt werden. Insbesondere können mehrere akustische Sensoren separat an unterschiedlichen Orten in dem Körper des Patienten positioniert werden, beispielsweise in unterschiedlichen Abschnitten des Rachens und/oder der Luftröhre. Die zwei oder mehr akustischen Sensoren werden bevorzugt in zwei in axialer Richtung hintereinander angeordneten Manschetten eingesetzt. Somit können die detektierten Signale der mehreren Sensoren, welche beispielsweise durch einen definierten axialen Abstand entlang des Beatmungstubus voneinander beabstandet sind, miteinander verglichen werden. Dies kann die Zuverlässigkeit der detektierten Signale erhöhen.

Wie zuvor bereits angemerkt, können der erste akustische Sensor und der zweite akustische Sensor auch in diesem Fall eine gemeinsame Schnittstelle aufweisen. Die detektierten Signale der zwei oder mehr akustischen Sensoren können somit zunächst zu der gemeinsamen Schnittstelle geleitet werden und dann gemeinsam an eine Recheneinrichtung übermittelt werden. Dies stellt eine möglichst kurze elektrische Kabelführung innerhalb des Beatmungstubus sicher. Die gemeinsame Schnittstelle ist bevorzugt auf einer Platine angeordnet, welcher die zwei oder mehr akustischen Sensoren oder noch weitere, andere Sensoren zugeordnet sind. Somit lässt sich besonders einfach ein möglichst kleines Mikrosystem mit mehreren Sensoren und einer gemeinsamen Schnittstelle realisieren.

Für sehr kleine Luftröhren wie z.B. bei Kindern oder kleineren Tieren werden Beatmungsschläuche ohne Manschette eingesetzt. Hier kann die Sensorik so aufgeführt sein, dass Sie in die Wand der Minituben integriert ist. Dies ist u.a. deshalb so ausgebildet, damit das innere Lumen zum Transport der Luft möglichst nicht eingeengt wird bzw. die sehr empfindliche Luftröhre keine Druckschäden erfährt. Der Sensor oder die Sensoren können so ausgebildet sein, dass eine Membran an der Innenwand des Tubus das darunter in der Wand eingebrachte Mikrofon überkleidet, wobei das Mikrofon eine angepasste Luft - bzw. Gasblase umgibt. Auf diese Weise ließe sich auch bei den hier beschriebenen größeren Beatmungstuben mit Manschette eine weitere Sensorik einbringen.

Neben dem zuvor erläuterten Beatmungstubus wird mit der Erfindung ein System aus einem solchen Beatmungstubus und einer Recheneinrichtung vorgeschlagen, wobei die Recheneinrichtung ausgebildet ist, detektierte Signale des in dem Luftvolumen der Manschette des Beatmungstubus angeordneten akustischen Sensors auszuwerten. Erfindungsgemäß werden die mittels des einen oder mehrerer akustischer Sensoren detektierten Signale an eine Recheneinrichtung des Systems weitergeleitet. Die detektierten Signale des akustischen Sensors beziehungsweise der akustischen Sensoren oder anderer Sensoren, welche physiologische Parameter des Patienten aufnehmen, können beispielsweise über eine Schnittstelle gesammelt als ein Datensatz an die Recheneinrichtung übermittelt werden. Die Recheneinrichtung, insbesondere ein Computer mit dafür speziell ausgebildeter Software, kann den Datensatz analysieren und die darin enthaltenen Parameter des Patienten extrahieren oder analysieren.

Das System kann bevorzugt eine Speichereinrichtung aufweisen, welche eine Vielzahl von Referenzdaten aufweist, wobei die Recheneinrichtung eingerichtet ist, die detektierten Signale des akustischen Sensors, und gegebenenfalls zusätzlicher Sensoren, mit den in der Speichereinrichtung gespeicherten Referenzdaten zu vergleichen. Die Referenzdaten können zeitlich früher aufgenommene Daten desselben Patienten oder Referenzdaten, welche anhand einer Vielzahl von physiologischen Daten mehrerer Patienten ermittelt und errechnet wurden, sein. Der Abgleich mit einer Vielzahl von Patientendaten ermöglicht eine bessere Analyse und Diagnose. Insbesondere kann anhand von Mustererkennung und Klassifizierung von medizinischen Daten die Diagnostik verbessert werden. Es können somit unter anderem medizinische Besonderheiten des individuellen Patienten erkannt werden, die nur im Rahmen einer komplexen Analyse einer großen Vielzahl von Messdaten unterschiedlicher Individuen auffindbar sind.

Eine derartige komplexe Analyse anhand einer Datenmenge sehr vieler Individuen kann sowohl in Bezug auf die Messdaten des akustischen Sensors, das heißt detektierte Signale betreffend die Atmung oder Beatmung des Patienten, als auch in Bezug auf detektierte Signale anderer Sensoren, die andere physiologische Parameter betreffen, beispielsweise Temperatur, Herzfrequenz, Blutfluss und andere, durchgeführt werden. Es ergibt sich somit ein komplexes Datenprofil des Patienten, welches mit ebensolchen komplexen Datenprofilen anderer Individuen verglichen werden kann. Je mehr Datensätze zu der Datenmenge beitragen, desto höher ist die Wahrscheinlichkeit, dass das Datenprofil des Patienten zu einem bereits gespeicherten Referenzprofil korrespondiert oder diesem zumindest so stark ähnelt, dass eine sichere Diagnose getroffen werden kann.

Schließlich wird vorgeschlagen, dass die Recheneinrichtung eingerichtet ist, in Abhängigkeit von dem Vergleichsergebnis eine Behandlungsempfehlung an einen Nutzer des Systems auszugeben und/oder ein in Kommunikationsverbindung mit der Recheneinrichtung stehendes medizinisches Gerät, insbesondere eine Beatmungseinrichtung, zu steuern. Die Ausgabe des Vergleichsergebnisses, insbesondere an medizinisches Personal oder einen Arzt, kann unter anderem in Form von Bildern, Symbolen, Text oder Ton erfolgen. Insbesondere kann ein Schwellwertvergleich vorgesehen sein, sodass der Nutzer erst dann über ein bestimmtes Vergleichsergebnis informiert wird, wenn die oder der betreffende Parameter einen zuvor definierten Schwellwert über- oder unterschreitet. Des Weiteren kann, insbesondere ohne weiteres Zutun des Nutzers, ein in Kommunikationsverbindung mit der Recheneinrichtung stehendes medizinisches Gerät automatisch gesteuert werden. Beispielsweise kann bei der Identifizierung von Schleim innerhalb der Atemwege des Patienten automatisch eine Pumpe angesteuert werden, welche den Schleim über eine entsprechende Strömungsleitung des Beatmungstubus absaugt. Des Weiteren kann die Pumpeinrichtung, welche die Manschette aufpumpt und somit für eine optimale Anlage des Beatmungstubus an der Luftröhre des Patienten dient, automatisch in Abhängigkeit von dem Vergleichsergebnis gesteuert werden. Damit kann fortlaufend der Druck in der Manschette dem aktuellen Beatmungsdruck angepasst werden und dabei die Schleimhaut der Luftröhre bestmöglichst druckentlastet werden. Nicht zuletzt kann die Beatmungseinrichtung, welche den Patienten über den Beatmungstubus beatmet, in Echtzeit von Atemzug zu Atemzug in einem geschlossenen Regelkreis ideal gesteuert werden und so komplexe Beatmungsschemata abbilden. Eine ebensolche Vorgehensweise ergibt sich für weitere medizinische Geräte, welche andere Funktionen steuern und nicht nur die Atmung beziehungsweise Beatmung des Patienten betreffen. Insbesondere können dies Geräte sein, welche automatisch Medikamente innerhalb des Patienten freisetzen oder dessen Blutdruck oder anderes steuern.

Insgesamt ergibt sich somit ein System aus einem Beatmungstubus und einer Recheneinrichtung, welches die Diagnostik der Körperparameter des Patienten deutlich gegenüber dem Stand der Technik verbessert, die Eingriffe in den Körper des Patienten reduziert und dementsprechend den Organismus weniger belastet. Eine erweiterte Sensorik kann des Weiteren über entstehende Redundanzen die Qualität der schlussendlich detektierten Informationen weiter verbessern. Moderne komplexe Beatmungsmuster können aufgrund der hier erweiterten Sensorik automatisiert noch tiefergehend abgebildet werden. Angelernte Algorithmen können so selbststeuernd noch viel besser den therapeutischen Erfolg, insbesondere bei schwierigen und komplexen Beatmungssituationen, in Echtzeit sicherstellen.

Vorteilhafte Weiterbildungen der Erfindung ergeben sich aus den Patentansprüchen, der Beschreibung und den Zeichnungen.

Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen.

Hinsichtlich des Offenbarungsgehalts - nicht des Schutzbereichs - der ursprünglichen Anmeldungsunterlagen und des Patents gilt Folgendes: Weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen, was aber nicht für die unabhängigen Patentansprüche des erteilten Patents gilt.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Sensor oder einer Manschette die Rede ist, ist dies so zu verstehen, dass genau ein Sensor oder eine Manschette, zwei Sensoren oder zwei Manschetten oder noch mehr Sensoren oder Manschetten vorhanden sind. Die in den Patentansprüchen angeführten Merkmale können durch weitere Merkmale ergänzt werden oder die einzigen Merkmale sein, die der Gegenstand des jeweiligen Patentanspruchs aufweist.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: zeigt ein mögliches Ausführungsbeispiel eines Beatmungstubus mit einer Manschette sowie einer zum Aufpumpen der Manschette verwendbaren Pumpeinrichtung.
- **Fig. 2**: zeigt ein System aus einem Beatmungstubus und einer Recheneinrichtung.
- **Fig. 3**: zeigt einen Beatmungstubus gemäß einem weiteren Ausführungsbeispiel.
- **Fig. 4**: zeigt einen Querschnitt durch einen Beatmungstubus im Bereich einer Manschette.
- **Fig. 5**: zeigt einen vergrößerten Querschnitt durch einen Beatmungstubus im Bereich eines akustischen Sensors.
- **Fig. 6**: zeigt einen vergrößerten Teilausschnitt des Querschnitts gemäß Fig. 5.
- **Fig. 7**: zeigt einen Beatmungstubus gemäß einem weiteren Ausführungsbeispiel.

### FIGURENBESCHREIBUNG

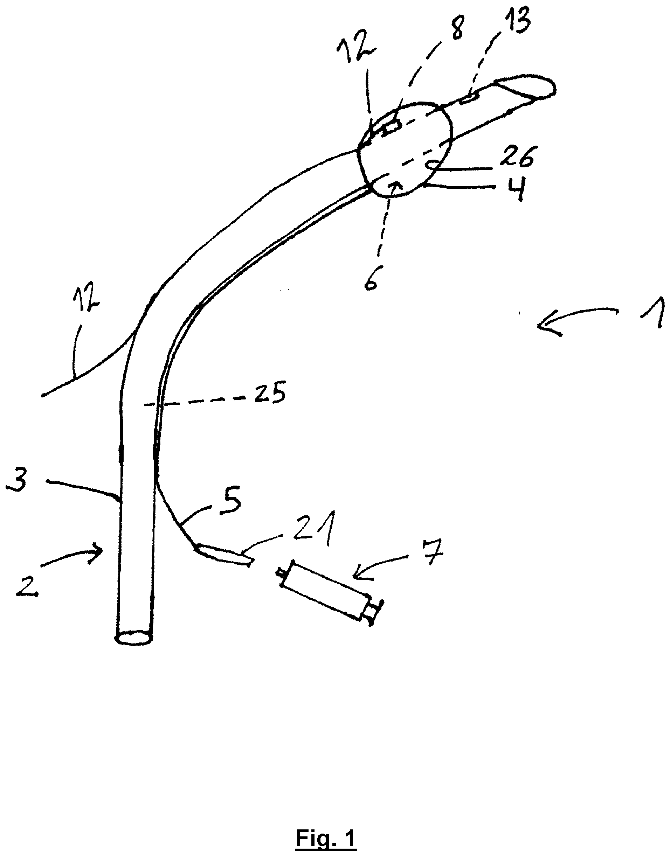

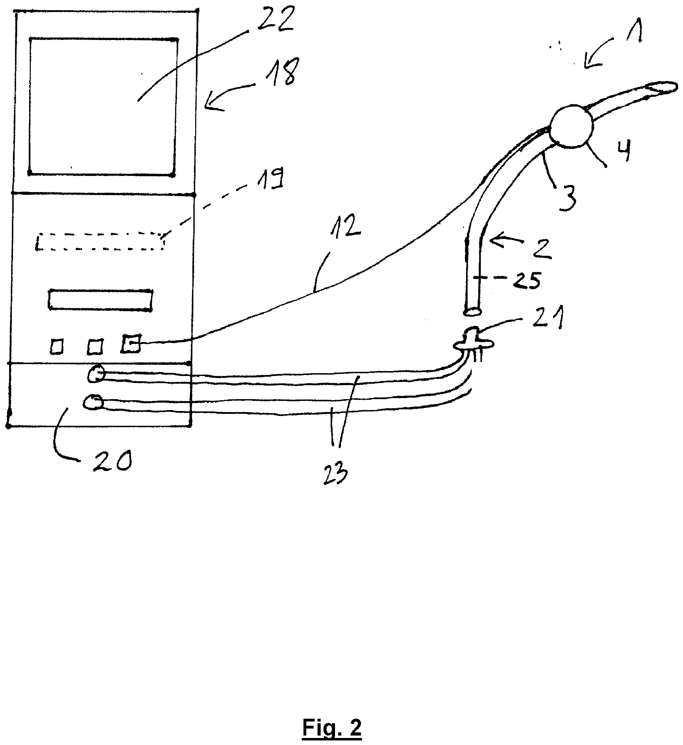

**Fig. 1** zeigt eine exemplarische Ausführungsform eines Beatmungstubus 1, welcher zum Beatmen eines Patienten beispielsweise während einer Operation oder in einer Notfallsituation verwendet wird. Der Beatmungstubus 1 ist als Endotrachealtubus ausgebildet und weist einen Schlauch 2 mit einer Schlauchwandung 3 auf. Der Schlauch 2 kann an korrespondierende Schlauchleitungen 23 bzw. ein Ventil 21 eines medizinischen Gerätes 20 wie beispielsweise einen manuell betätigbaren Beatmungsbeutel oder ein automatisches Beatmungsgerät angeschlossen werden (siehe Figur 2). Über die Schlauchleitungen 23 des medizinischen Gerätes 20 gelangt Luft in den Beatmungstubus 1 und damit schließlich in die Lunge des Patienten. An dem medizinischen Gerät 20 kann dabei eingestellt werden, wie die automatische Beatmung ablaufen soll, insbesondere wie lange ein Atemzug dauern soll, wieviel Luft dabei in die Lunge strömt, welcher Druck in den Atemwegen herrscht und/oder wie hoch der Sauerstoffgehalt der Luft sein soll. Zudem lassen sich der Atemluft Narkosegase oder Feuchtigkeit beimischen. Über eine Anzeigeeinrichtung 22, beispielsweise einen Monitor, kann sich medizinisches Personal oder ein Arzt alle wesentlichen Patientenparameter anzeigen lassen.

Da während der Beatmung das Risiko besteht, dass Mageninhalt über die Speiseröhre in den Mund-Rachen-Raum zurückfließt und von dort in die Luftröhre gelangt, ist der Beatmungstubus 1 mit einer Manschette 4 ausgestattet, welche einen axialen Abschnitt der Schlauchwandung 3 des Schlauches 2 umgibt. Die Manschette 4 ist über eine Pumpleitung 5 aufpumpbar. Das Aufpumpen der Manschette 4 erfolgt üblicherweise manuell mittels einer Pumpeinrichtung 7, welche eine einfache Spritze mit einem Fassungsvermögen von mehreren Millilitern (beispielsweise 10ml) sein kann. Darüber hinaus sind sogenannte Blockerspritzen bekannt, welche nach der Betätigung dem aufgebauten Gegendruck des Beatmungstubus 1 standhalten. Die Pumpleitung 5 des Beatmungstubus 1 wird üblicherweise außen an der Schlauchwandung 3 des Beatmungstubus 1 entlanggeführt bis in ein Inneres der Manschette 4. Durch das Aufpumpen baut sich zwischen der Schlauchwandung 3 und der Innenwandung der Manschette 4 ein Luftvolumen 6 auf. Durch dieses Luftvolumen 6 drückt die Manschette 4 von innen gegen die Luftröhre des Patienten. Die Manschette 4 fixiert den Schlauch 2 und dichtet die Luftröhre ab. Somit kann kein Mageninhalt an dem Beatmungstubus 1 vorbei in die Luftröhre fließen. Bevor der Beatmungstubus 1 nach dem Eingriff wieder entfernt wird, wird die Luft aus der Manschette 4 abgelassen.

Wie in Fig. 1 des Weiteren dargestellt, wird das Luftvolumen 6 zwischen der Schlauwandung 3 und der Manschette 4 dazu verwendet, einen akustischen Sensor 8 aufzunehmen, welcher in dem dargestellten expandierten Zustand der Manschette 4 durch das Luftvolumen 6 von einer gegenüberliegenden Innenwandung 26 der Manschette 4 beabstandet ist und lediglich Kontakt zu der Schlauchwandung 3 des Schlauches 2 aufweist. Der akustische Sensor 8 ist hier vorzugsweise ein Mikrofon, welches insbesondere Atemgeräusche beziehungsweise Beatmungsgeräusche, die im Bereich des Schlauches 2 wahrnehmbar sind, detektiert. Vorteilhaft ist dabei, dass der akustische Sensor 8 von der äußeren Umfangsfläche der Manschette 4 beabstandet ist und somit vorrangig nicht sonstige Körpergeräusche wie beispielsweise einen Herzschlag des Patienten detektiert, sondern vielmehr hauptsächlich Atemgeräusche oder Beatmungsgeräusche, die durch die in dem Schlauch 2 geführte Luft übertragen werden. Charakteristische Geräusche werden beispielsweise verursacht durch regelmäßige Atmung beziehungsweise Beatmung, Apnoe, Husten oder die Bildung von Schleim innerhalb der Atemwege. Die akustischen Signale aus der Luftröhre des Patienten liefern somit zahlreiche Informationen über den aktuellen Zustand des Patienten und können als Indikation für eine nachfolgende Behandlung dienen beziehungsweise für eine weiterfolgende Einstellung der automatischen Beatmung.

Die Messdaten, d.h. die detektierten Signale, können in ein Dokumentationssystem einfließen und mittels einer in **Fig. 2** dargestellten Recheneinrichtung 18 analysiert werden, indem die aktuellen Messdaten mit in einer Speichereinrichtung 19 gespeicherten Referenzdaten verglichen werden. Die Referenzdaten können zusammengetragene Metadaten einer großen Vielzahl von bisher behandelten Patienten sein. Sofern der analysierte Zustand eines Patienten einem bestimmten Datensatz innerhalb der Speichereinrichtung 19 entspricht oder ähnelt, kann auf den aktuellen Zustand des Patienten geschlossen werden und eine entsprechende Maßnahme ergriffen werden. Eine solche Maßnahme kann beispielsweise die Entfernung von Schleim aus den Atemwegen sein. Die zu ergreifende Maßnahme kann entweder automatisch durch die Recheneinrichtung 18 gesteuert werden oder zunächst einem behandelnden Arzt oder medizinischem Fachpersonal auf einer Anzeigeeinrichtung 22 vorgeschlagen bzw. angezeigt werden.

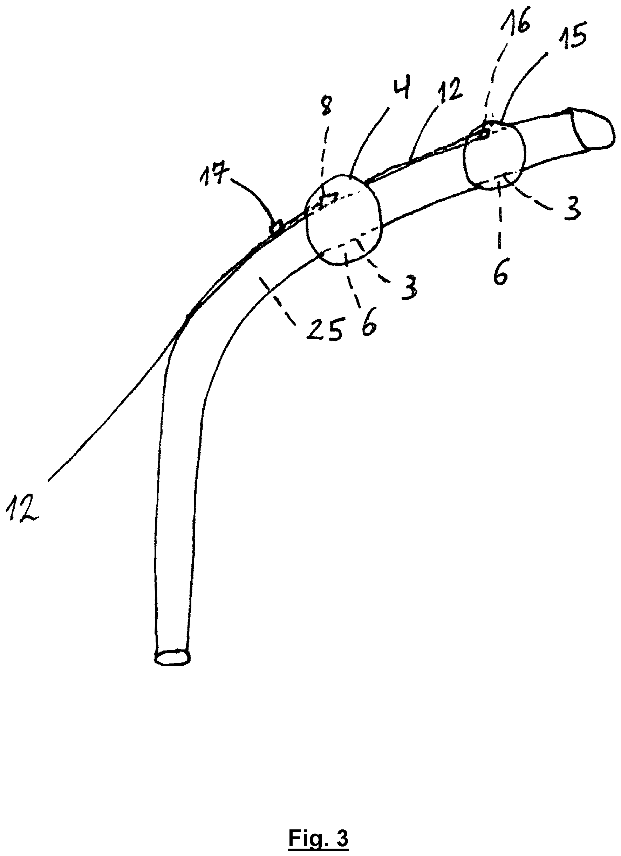

Die **Fig. 3** zeigt ein weiteres alternatives Ausführungsbeispiel eines Beatmungstubus 1 mit zwei in axialer Richtung des Schlauches 2 aufeinanderfolgenden Manschetten 4, 15, welche mit einem bestimmten axialen Abstand in Längserstreckungsrichtung des Schlauches 2 angeordnet sind. Jede der Manschetten 4, 15 ist wie zuvor dargestellt über eine Pumpleitung 5 aufpumpbar (in Fig. 3 nicht näher dargestellt). Jede der Manschetten 4, 15 weist zu der Schlauchwandung 3 ein Luftvolumen 6 auf, in welchem ein akustischer Sensor 8, 16 platziert ist. Die elektrischen Leitungen 12 der akustischen Sensoren 8, 16 sind über eine gemeinsame Schnittstelle 17 miteinander verbunden, sodass deren detektierte Signale über eine gemeinsame elektrische Leitung 12 an die Recheneinrichtung 18 übermittelt werden können. Dies verhindert, dass eine Vielzahl von separaten elektrischen Leitungen 12 an dem Beatmungstubus 1 erforderlich ist.

Wie in den Fig. 1 bis 3 dargestellt, können die elektrischen Leitungen 12 außen an der Schlauchwandung 3 angeordnet sein. Jedoch wäre es alternativ möglich, dass die elektrischen Leitungen 12 in die Schlauchwandung 3 integriert sind. Es können auch weitere Leitungen in die Schlauchwandung 3 integriert werden, beispielsweise die Pumpleitung 5 zum Aufpumpen der Manschette 4 beziehungsweise der Manschetten 4, 15.

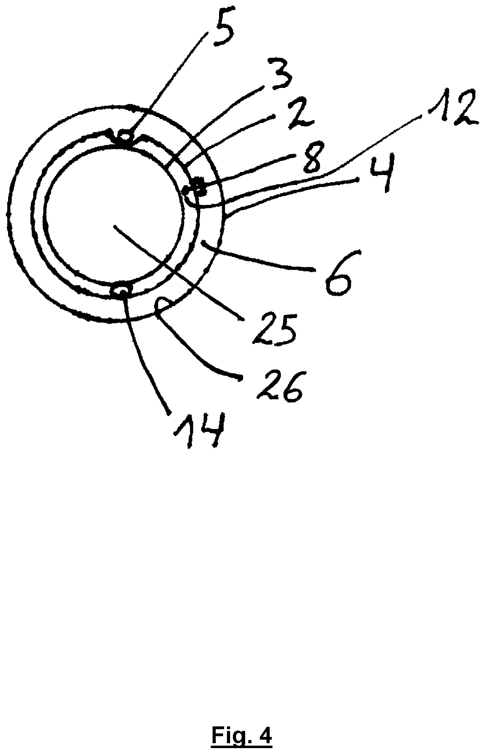

Der Schlauch 2 kann des Weiteren - wie beispielsweise in dem Querschnitt gemäß **Fig. 4** gezeigt - eine in Axialrichtung verlaufende Strömungsleitung 14 zum Absaugen von Körperflüssigkeiten oder zum Spülen von Körperparteien aufweisen. Dadurch kann beispielsweise in den Atemwegen befindlicher Schleim abgesaugt oder weggespült werden. Auch diese Strömungsleitung 14 kann in die Schlauchwandung 3 integriert sein. Alternativ wäre es möglich, diese Strömungsleitung 14 außerhalb der Schlauchwandung 3 zu platzieren.

Die Fig. 4 zeigt des Weiteren einen akustischen Sensor 8 sowie eine Pumpleitung 5 zum Aufpumpen der Manschette 4. Sowohl die Pumpleitung 5, als auch die Strömungsleitung 14 sind hier beispielsweise in die Schlauchwandung 3 des Schlauches 2 integriert, wobei durch die Pumpleitung 5 strömende Luft in radialer Richtung aus der Schlauchwandung 3 in die Manschette 4 übertreten kann. In dem Luftvolumen 6, welches zwischen der Manschette 4 und der Schlauchwandung 3 ausgebildet ist, befindet sich außen an der Schlauchwandung 3 der akustische Sensor 8. Bei expandierter Manschette 4 weist dieser akustische Sensor 8 wie dargestellt keinen Kontakt zu der Innenwandung der Manschette 4 auf.

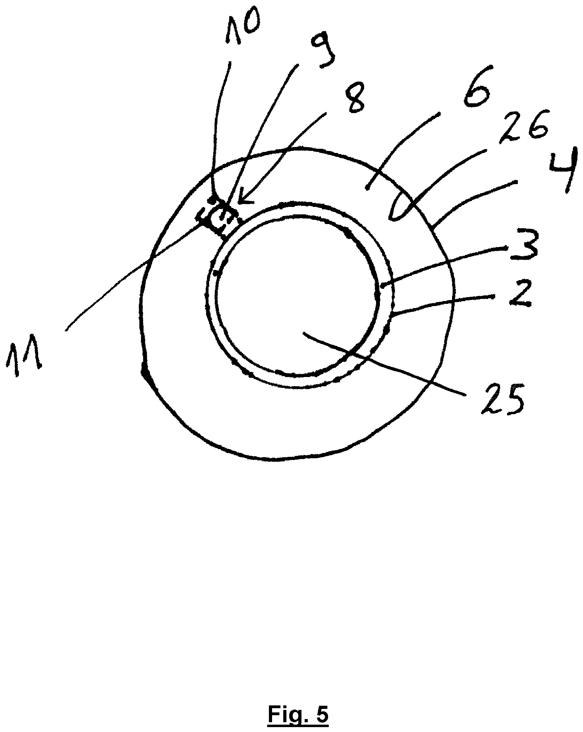

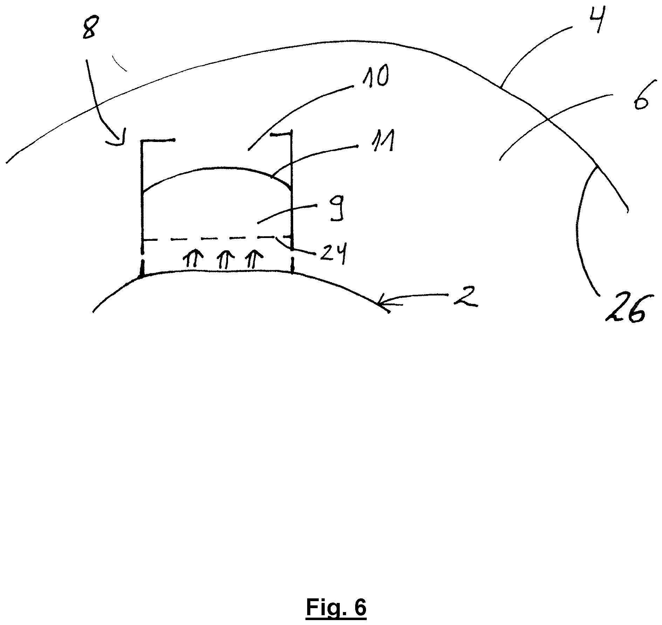

Der akustische Sensor 8 ist des Weiteren in den **Fig. 5** und **6** vergrößert dargestellt. Der akustische Sensor 8 ist hier bevorzugt ein MEMS-Mikrofon, welches als Komponente eines Mikrosystems ausgebildet ist. MEMS-Mikrofone sind bereits aus einer Vielzahl von elektronischen Produkten bekannt, wie beispielsweise Mobiltelefone, Tablet- und Laptop-Computer sowie andere Smart-Geräte.

Ein solches MEMS-Mikrofon als hier bevorzugte Ausführung eines akustischen Sensors 8 besteht im Wesentlichen aus einer ersten Luftkammer 9, einer zweiten Luftkammer 10 und einer dazwischen angeordneten Membran 11, welche sich bei angreifendem Schalldruck (in Fig. 6 dargestellt durch Pfeile) verformt (krümmt) und somit eine Kapazität zwischen einer feststehenden Basisplatte 24 des Sensors 8 und der Membran 11 ändert. Die Änderung der Kapazität erzeugt ein messbares elektronisches Signal an einer zugeordneten Elektrode.

In dem Luftvolumen 6 zwischen der Manschette 4 (beziehungsweise der weiteren Manschette 15 gemäß Fig. 3) und der Schlauwandung 3 ist der akustische Sensor 8 (beziehungsweise der akustische Sensor 16) so angeordnet, dass sowohl die erste Luftkammer 9, als auch die zweite Luftkammer 10 eine Strömungsverbindung zu dem Luftvolumen 6 aufweist. Dadurch ist sichergestellt, dass die Membran 11 des akustischen Sensors 8 bzw. 16 auf ihren beiden Seiten dem in dem Luftvolumen 6 herrschenden Überdruck, aber keinem Differenzdruck aufgrund dieses Überdrucks ausgesetzt ist. Dies sorgt dafür, dass die Membran 11 durch den Überdruck nicht statisch ausgelenkt wird, so dass sich die Membran 11 bei auftretendem Schalldruck, der typischerweise viel kleiner als der Überdruck ist, bewegen kann.

Des Weiteren kann es vorgesehen sein, dass der Beatmungstubus 1 unabhängig von dem akustischen Sensor 8, 16 einen weiteren Sensor 13 oder mehrere weitere Sensoren 13 aufweist, die nicht unbedingt der Manschette 4, 15 des Beatmungstubus 1 zugeordnet sein müssen, sondern vielmehr außerhalb der Manschette 4, 15 auf der Schlauchwandung 3 platziert oder in die Schlauchwandung 3 integriert sein können. In Fig. 1 ist beispielhaft ein einzelner solcher Sensor 13 dargestellt, welcher zur Messung eines oder mehrerer weiterer physiologischer Parameter des Patienten dient. Diese physiologischen Parameter können Parameter sein, welche insbesondere nicht Parameter der Atmung des Patienten oder der künstlichen Beatmung sind, sondern vielmehr einen Blutfluss und/oder eine Herzfrequenz und/oder einen Blutdruck und/oder eine Temperatur und/oder einen anderen Druck innerhalb des Körpers des Patienten betreffen. Dadurch, dass derartige zusätzliche Sensoren 13 nicht von dem Luftvolumen 6 zwischen der Manschette 4, 15 und der Schlauchwandung 3 umschlossen sind, lassen sich damit insbesondere sonstige Körpergeräusche detektieren, wie beispielsweise Geräusche die durch eine Darmtätigung entstehen. Insbesondere können so Darmgeräusche, Strömungsgeräusche in Blutgefäßen, Geräusche, die in kranken Gelenken entstehen, oder andere Körpergeräusche detektiert und analysiert werden. Die detektierten Signale sowohl des akustischen Sensors 8 oder der akustischen Sensoren 8, 16, als auch des einen weiteren Sensors 13 oder der mehreren weiteren Sensoren 13 können über nachgeschaltete computergestützte Analyse ausgewertet werden. Als Beispiel seien akustische Informationen aus der Luftröhre und Darmgeräusche genannt, die zahlreiche Informationen liefern und als Indikation für die weitere Behandlung dienen können. Die Messdaten können in der Recheneinrichtung 18 analysiert und mit Vergleichsdaten der Speichereinrichtung 19 verglichen werden, um Krankheiten beziehungsweise kritische Zustände des Patienten zu erkennen und bei Bedarf eine Behandlung einleiten zu können. Unter Umständen, insbesondere während einer Operation, können Missstände bereits sofort behoben werden. Dies ist insbesondere der Fall, wenn in den Atemwegen des Patienten Schleim entsteht. Dieser Schleim kann dann mittels eines externen medizinischen Gerätes 20 über die Strömungsleitung 14 abgesaugt werden.

Durch den Einsatz mehrerer akustischer Sensoren 8, insbesondere in separaten Manschetten 4, 15, kann eine Mehrkanalauflösung erreicht werden, was die Qualität der akustischen Signale erhöht und somit die Diagnose zuverlässiger gestaltet. Die akustischen Signale der akustischen Sensoren 8, 16 sowie der weiteren Sensoren 13 können zur Erstellung einer Geräusch-Referenz-Datenbank dienen. Dabei kann insbesondere eine künstliche Intelligenz angelernt werden, um akustische Signale auszuwerten und diesen bestimmte Krankheitsbilder zuzuordnen. Des Weiteren können derartige Geräusche nicht nur für einen einzigen Patienten detektiert und ausgewertet werden, sondern bezüglich einer großen Vielzahl von Individuen, sodass eine ausreichende Datenmenge gegeben ist, um auch seltene Krankheiten oder Beeinträchtigungen detektieren und zuordnen zu können.

In Abhängigkeit von dem Vergleichsergebnis zwischen einem aktuellen detektierten Signal und den innerhalb der Speichereinrichtung 19 gespeicherten Referenzdaten kann dann gegebenenfalls eine automatisierte Nachricht an einen Nutzer des Systems ausgegeben werden, insbesondere an medizinisches Personal oder einen Arzt. Die Ausgabe derartiger Informationen kann über eine Anzeigeeinrichtung 22 wie einen Monitor erfolgen. Jedoch ist es auch möglich, beispielsweise akustische Signale auszugeben. Des Weiteren kann vorgesehen sein, dass erst dann eine Information ausgegeben wird, wenn ein detektierter Parameter einen vordefinierten Schwellwert überschreitet oder unterschreitet und somit eine Notwendigkeit für ein Handeln gegeben ist. Darüber hinaus kann die Recheneinrichtung 18 in Abhängigkeit von dem Vergleichsergebnis automatisch ein medizinisches Gerät 20 steuern. Dieses medizinische Gerät 20 kann beispielsweise ein Beatmungsgerät sein, welches Frischluft in den Beatmungstubus 1 einleitet beziehungsweise verbrauchte Luft absaugt. Neben dem Beatmungsgerät an sich können andere medizinische Geräte 20 angesteuert werden, beispielsweise eine Heizung oder Kühlung, eine Pumpe zum Einbringen von Medikamenten in den Körper des Patienten oder zum Freisetzen von Medikamenten, welche über ein entsprechendes Bauteil bereits in dem Körper des Patienten platziert sind.

Insgesamt hat sich bei Versuchen gezeigt, dass es durch die erfindungsgemäße Platzierung eines akustischen Sensors 8, 16 an dem Beatmungstubus 1 möglich ist, in unmittelbarer Nähe der Aufteilung zwischen zwei Hauptbronchien der Lunge die dem Atem beziehungsweise der Beatmung des Patienten zugehörigen Parameter zuverlässig zu detektieren. Dabei zeigte sich, dass es sehr gut gelingt, zwischen normalen und gestörten Luftströmungsmustern zu unterscheiden, beispielsweise Schleimbildung, Spastik, Stridor und andere. Die digitalen Audio-Profile verschiedener Atmungszustände beziehungsweise Beatmungszustände können dabei ausreichend gut unterschieden werden. Insbesondere reicht es bereits aus, einen kurzen, zwei Atemzyklen umfassenden Datensatz zu detektieren und zu analysieren, um zwischen den Zuständen eines ordnungsgemäß beziehungsweise nicht ordnungsgemäß platzierten Beatmungstubus 1 zu unterscheiden. Des Weiteren kann dünn- oder dickflüssiger Schleim identifiziert werden, ein Pendelvolumen, Husten, eine maschinelle oder spontane Atmung. Mit der dabei angewendeten künstlichen Intelligenz lassen sich Änderungen von automatisierten Beatmungsvorgängen in bisher nicht möglicher Form einführen, nämlich Beatmungsanpassungen, Absaugvorgänge oder andere. Die verwendeten Algorithmen zur Analyse können auf kostengünstigen Minicomputern laufen oder als Cloudlösung ausgeführt sein.

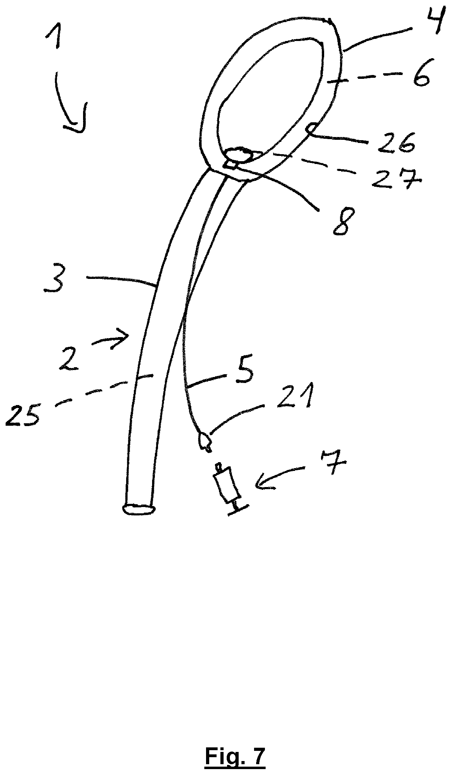

Die Fig. 7 zeigt eine andere Ausführungsform eines Beatmungstubus 1. Dieser Beatmungstubus 1 ist als Kehlkopfmaske ausgebildet, welche im Rachen eines Patienten vor den Kehlkopf geschoben wird, um dort die Luftröhre abzudichten. Dieser Beatmungstubus 1 weist eine endseitige Manschette 4 auf, welche eine Austrittsöffnung 27 des Schlauches 2 des Beatmungstubus 1 ohrmuschelförmig umgibt und dabei, bezogen auf einen Querschnitt orthogonal zu der Längserstreckung des Luftkanals 25, ringförmig an die Stirnseite des Schlauches 2 anschließt. Dadurch ergibt sich eine Verlängerung des in dem Schlauch 2 ausgebildeten Luftkanals 25 durch die Manschette 4. Die zumindest im Bereich der Austrittsöffnung 27 des Schlauches 2 doppelwandig ausgebildete Manschette 4 ist mittels einer Pumpeinrichtung 7 aufblasbar, wodurch sich ein Luftvolumen 6 innerhalb der Manschette 4 ausweitet und diese gegen den Kehlkopf des Patienten drückt.

In dem Luftvolumen 6 der Manschette 4 befindet sich, mit Abstand zu einer radial äußeren Innenwandung 26 der Manschette 4, ein akustischer Sensor 8 auf einem an den Luftkanal 25 angrenzenden Teilbereich der Manschette 4. Damit ist der akustische Sensor 8 durch das Luftvolumen 6 von der radial äußeren Innenwandung 26 getrennt und kann vorrangig im Bereich des Luftkanals 25 Geräusche detektieren, insbesondere solche, die sich über den Luftkanal 25 des Beatmungstubus 1 verbreiten. Insbesondere sind dies Atemgeräusche des Patienten und Beatmungsgeräusche, welche durch die künstliche Beatmung entstehen. Andere Körpergeräusche, wie beispielsweise solche, die den Herzschlag des Patienten betreffen, sind durch den akustischen Sensor 8 hingegen weniger wahrnehmbar, da der akustische Sensor 8 durch das in der Manschette 4 ausgebildete Luftvolumen 6 von der radial äußeren Innenwandung 26 getrennt ist.

Im Übrigen gelten, unter anderem in Bezug auf die Detektion und Analyse der von dem akustischen Sensor 8 detektierten Signale, die Ausführungen zu den vorausgehenden Ausführungsbeispielen der Figuren 1 bis 6. Des Weiteren kann die in Fig. 7 beispielhaft dargestellte Kehlkopfmaske auch einen oder mehrere zusätzliche Sensoren 13 (in Fig. 7 nicht dargestellt) aufweisen, um insbesondere andere physiologische Parameter des Patienten zu detektieren.

### BEZUGSZEICHENLISTE

- 1: Beatmungstubus
- 2: Schlauch
- 3: Schlauchwandung
- 4: Manschette
- 5: Pumpleitung
- 6: Luftvolumen
- 7: Pumpeinrichtung
- 8: akustischer Sensor
- 9: erste Luftkammer
- 10: zweite Luftkammer
- 11: Membran
- 12: elektrische Leitung
- 13: Sensor
- 14: Strömungsleitung
- 15: Manschette
- 16: akustischer Sensor
- 17: Schnittstelle
- 18: Recheneinrichtung
- 19: Speichereinrichtung
- 20: medizinisches Gerät
- 21: Ventil
- 22: Anzeigeeinrichtung
- 23: Schlauchleitung
- 24: Basisplatte
- 25: Luftkanal
- 26: Innenwandung
- 27: Austrittsöffnung

## Patentansprüche

1. Beatmungstubus (1) zum Beatmen eines Patienten, aufweisend
- einen, einen Luftkanal (25) umgebenden Schlauch (2) mit einer Schlauchwandung (3) zum Einführen bis in einen Rachen oder bis in eine Luftröhre des Patienten,
- eine Manschette (4, 15) mit einem in Umfangsrichtung der Schlauchwandung (3) umlaufenden Luftvolumen (6),
- eine in Axialrichtung des Schlauches (2) verlaufende Pumpleitung (5) zum Herstellen einer Strömungsverbindung zwischen dem Luftvolumen (6) der Manschette (4, 15) und einer Pumpeinrichtung (7),
**gekennzeichnet durch** einen in dem Luftvolumen (6) auf der Schlauchwandung (3) oder einem an den Luftkanal (25) angrenzenden Teilbereich der Manschette (4, 15) angeordneten akustischen Sensor (8, 16).

2. Beatmungstubus (1) nach Anspruch 1, **dadurch gekennzeichnet, dass** der akustische Sensor (8, 16) zumindest in expandiertem Zustand der Manschette (4, 15) durch das Luftvolumen (6) von einer gegenüberliegenden radial äußeren Innenwandung (26) der Manschette (4, 15) beabstandet ist.

3. Beatmungstubus (1) nach einem der vorhergehenden Ansprüche, **dadurch gekennzeichnet, dass** der akustische Sensor (8, 16) ein Mikrofon, insbesondere ein als Komponente eines Mikrosystems ausgebildetes Mikrofon, ist.

4. Beatmungstubus (1) nach Anspruch 3, **dadurch gekennzeichnet, dass** das Mikrofon eine erste Luftkammer (9), eine zweite Luftkammer (10) und eine, die erste Luftkammer (9) von der zweiten Luftkammer (10) trennende flexible Membran (11) aufweist, wobei sowohl die erste Luftkammer (9), als auch die zweite Luftkammer (10) eine Strömungsverbindung zu dem Luftvolumen (6) aufweist.

5. Beatmungstubus (1) nach einem der vorhergehenden Ansprüche, **dadurch gekennzeichnet, dass** die Pumpleitung (5) in die Schlauchwandung (3) integriert ist.

6. Beatmungstubus (1) nach einem der vorhergehenden Ansprüche, **dadurch gekennzeichnet, dass** eine elektrische Leitung (12) für den akustischen Sensor (8, 16) in die Schlauchwandung (3) integriert ist.

7. Beatmungstubus (1) nach einem der vorhergehenden Ansprüche, **gekennzeichnet durch** einen oder mehrere weitere Sensoren (13) zur Detektion physiologischer Parameter, insbesondere zur Messung eines Blutflusses und/oder einer Herzfrequenz und/oder einer Atemfrequenz und/oder eines Blutdruckes und/oder einer Temperatur und/oder eines Druckes.

8. Beatmungstubus (1) nach einem der vorhergehenden Ansprüche, **dadurch gekennzeichnet, dass** der Schlauch (2) eine in Axialrichtung verlaufende Strömungsleitung (14) zum Absaugen von Körperflüssigkeiten oder zum Spülen von Körperpartien aufweist.

9. Beatmungstubus (1) nach Anspruch 8, **dadurch gekennzeichnet, dass** die Strömungsleitung (14) in die Schlauchwandung (3) integriert ist.

10. Beatmungstubus (1) nach einem der vorhergehenden Ansprüche, **gekennzeichnet durch** zwei, die Schlauchwandung (3) umgebende und voneinander beabstandete Manschetten (4, 15), wobei ein erster akustischer Sensor (8) einer ersten Manschette (4) zugeordnet ist und wobei ein zweiter akustischer Sensor einer zweiten Manschette (15) zugeordnet ist.

11. Beatmungstubus (1) nach Anspruch 10, **dadurch gekennzeichnet, dass** der erste akustische Sensor (8) und der zweite akustische Sensor (16) eine gemeinsame Schnittstelle (17) aufweisen.

12. System aus einem nach einem der vorhergehenden Ansprüche ausgebildeten Beatmungstubus (1) und einer Recheneinrichtung (18), wobei die Recheneinrichtung (18) ausgebildet ist, detektierte Signale des in dem Luftvolumen (6) der Manschette (4, 15) des Beatmungstubus (1) angeordneten akustischen Sensors (8, 16) auszuwerten.

13. System nach Anspruch 12, **gekennzeichnet durch** eine Speichereinrichtung (19), welche eine Vielzahl von Referenzdaten aufweist, wobei die Recheneinrichtung (18) eingerichtet ist, die detektierten Signale des akustischen Sensors (8, 16) mit den in der Speichereinrichtung (19) gespeicherten Referenzdaten zu vergleichen.

14. System nach Anspruch 12 oder 13, **dadurch gekennzeichnet, dass** die Recheneinrichtung (18) eingerichtet ist, in Abhängigkeit von dem Vergleichsergebnis eine Behandlungsempfehlung an einen Nutzer auszugeben und/oder ein in Kommunikationsverbindung mit der Recheneinrichtung (18) stehendes medizinisches Gerät (20), insbesondere eine Beatmungseinrichtung, zu steuern.
